# EUROPEAN PATENT SPECIFICATION

(11) **EP 0 707 837 B1**
(45) Date of publication and mention of the grant of the patent: **10.12.2003**
(21) Application number: 95202790.2
(22) Date of filing: 16.10.1995
(51) Int. Cl.: A61F 2/06, A61M 25/10

(54) **Catheter for stent implantation**
Katheter für Stentimplantation
Cathéter pour implantation d'un stent

(30) Priority: 20.10.1994 NL 9401744; 21.10.1994 NL 9401758; 15.02.1995 NL 9500284
(43) Date of publication of application: 24.04.1996
(73) Proprietor: CORDIS EUROPA N.V., NL-9301 LJ Roden (NL); Sheiban, Imad, 37126 Verona (IT)
(72) Inventor: Sheiban, Imad, I-37126 Verona (IT); Nap, Cornelis Philipus, NL-9345 AC Zevenhuizen (NL)
(74) Representative: 't Jong, Bastiaan Jacob

(56) References cited:
- EP-A- 0 274 846
- EP-A- 0 442 657
- EP-A- 0 528 039
- WO-A-87/15787
- US-A- 5 226 889

## Description

The invention relates to a catheter used for the implantion of a stent in a patient. A stent is a tubular element, usually made of wire mesh, which can be expanded in cross-sectional direction. Such an element is for instance introduced into a blood vessel of a patient, of which the wall has been impaired to such a degree, that there is a risk of collapse of the blood vessel concerned. The stent is introduced in a compressed state and expanded when situated in the required position, thus supporting the wall of the blood vessel. Expansion is usually achieved by means of the balloon of a balloon catheter. Stents and catheters employed for the introduction thereof are known as such, and are for instance described in the American patent specification 5 226 889, EP 0 274 846-A-1 and international patent application WO 93/15787.

WO 93/15787 shows a catheter as specified in the preamble of claim 1, namely with a tube-like body, at least one balloon arranged close to the distal end of the catheter and connected via a lumen with a connecting member at the proximal end of the catheter, wherein close to either ends of the balloon a ring has been arranged and a stent is arranged around the balloon and between the rings.

The object of this invention is to provide an improvement of known catheters used for the purpose of stent-implantation.

This aim has been achieved with the catheter according to the invention as characterised in claim 1. The rings can consequently be arranged relatively close to each other, without limiting the expansion possibilities of the balloon. Consequently the stent can be fixed properly in the center of the balloon. In the non-expanded state, the balloon fits closely over the rings, so that the surface of the catheter in a longitudinal direction over the rings is smooth. The rings are arranged in the balloon, so that no physical contact with the exterior can occur.

It is noted that EP 0 274 846-A-1 shows a catheter with a tube-like body, at least one balloon arranged close to the distal end of the catheter and connected via a lumen with a connecting member at the proximal end of the catheter, wherein close to either ends of the balloon a shoulder has been arranged and a stent is arranged around the balloon and between the bulges. The bulges have been arranged inside the balloon member. These bulges are not extending outwardly and are not formed by a separate member.

For a good fixation of the stent it is sufficient if, in relation to the inside diameter of the compressed stent, the rings are such that the stent cannot slide over these rings. Preferably however the measure as characterized in claim 2 is employed. The stent is, as it were, situated in a depression or "bed" defined by the rings, as a result of which the stent does not interfere with the introduction of the catheter.

The measure as set out in claim 3 is preferably employed. Both rings and therefore the position of the stent retained in between them, is in this way properly observable so that the stent can be manoeuvred carefully into the correct position.

Preferably the measure as set out in claim 5 is employed. The relatively distal balloon member can be used for dilatation of that section of the blood vessel where the stent is to be implanted, even when there is a severe stenosis. After dilatation of the blood vessel with the relatively distal balloon, the catheter is advanced a little further, thus positioning the stent in the dilated section of the blood vessel. Next the balloon around which the stent has been arranged can be expanded, as a result of which the stent will expand and provide the wall of the vessel with the required support. Thus, using one single catheter, and in one session, the vessel can be dilated and the stent implanted.

A suitable embodiment is characterised in claim 6.

The invention will be explained in greater detail in the following description with reference to the attached drawings.
- Figure 1: shows a partly broken away, perspective view of a catheter according to the invention.
- Figure 2: shows a partly cut away view of the distal end of the catheter of figure 1.
- Figure 3: shows the catheter section shown in figure 1 during a first step of the treatment for which it is to be used.
- Figure 4: shows a view corresponding to figure 3 of a second step of the treatment.
- Figure 5: shows a view corresponding to figure 3 and 4 of a third step of the treatment.
- Figure 6: shows schematically the treated blood vessel with the stent in position, following the removal of the catheter.
- Figure 7: shows a partly broken away, perspective view of a catheter according to another embodiment of the invention.

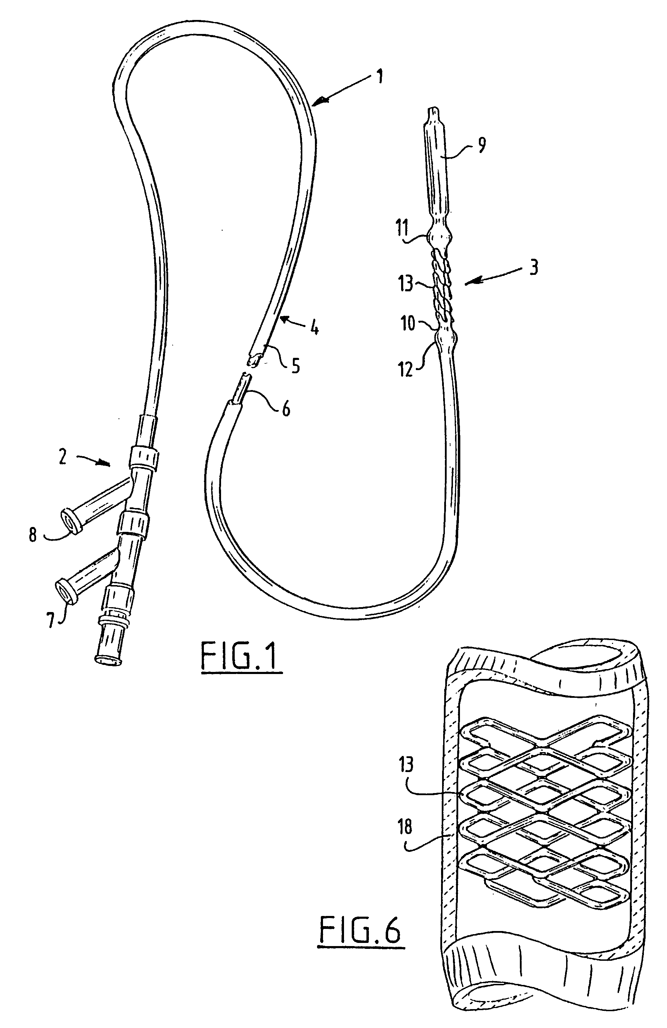

The catheter 1 shown in figure 1 comprises a tube-like basic body 4 which in this case is made up of an outer tube-like element 5 and an inner tube-like element 6 received inside a lumen thereof. The inner tub-like element 6 has two lumens 16, 17. With the embodiment shown, two balloon members 9, 10 have been arranged at the distal end 3 of the catheter 1. At the proximal end 2 two connecting members 7, 8 have been arranged which are connected to the balloon member 9 and the balloon member 10 respectively. By supplying medium under pressure via the connectors 7 or 8, the balloon members 9 or 10 can be ) expanded. The connection between the connecting member 7 and the balloon member 9 runs via lumen 17 of the inner tube-like element 6 and the connection between the connecting member 8 and the balloon member 10 runs via the interspace 20 in between the inner tube-like element 6 and the outer tube-like element 5. Lumen 16 is intended for receiving a guide wire.

On both sides of the balloon member 10, the catheter 1 has been provided with bulges 11, 12. A compressed stent 13 has been arranged around the balloon 10 in between those bulges 11 and 12. The stent 13 is enclosed by the bulges 11 and 12 in the axial direction of the catheter 1, so that it cannot slide off the balloon 10.

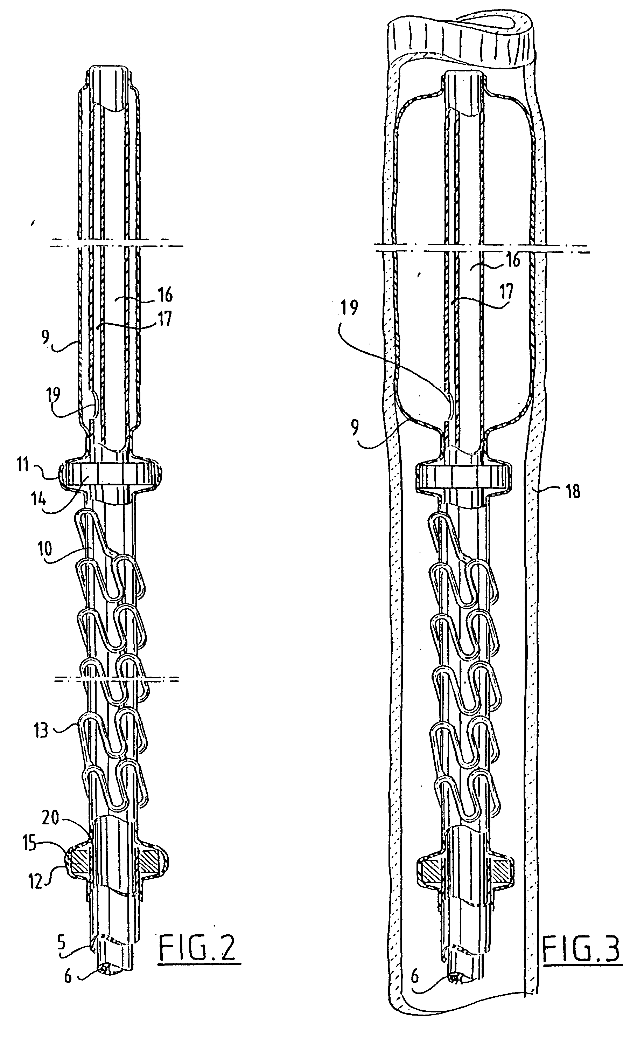

As can be seen in figure 2 et seq. in particular, the bulges 11 and 12 are formed by respective rings 14 and 15 which have been received inside the balloon 10. The ring 14 has been arranged around the continuous inner tube-like element 6 and the ring 15 has been arranged around the end of the outer tube-like element 5. As the rings 14, 15 have been arranged inside the balloon 10 they do not interfere with the unfolding of the balloon 10, and the balloon forms a smooth "skin" over the rings 14, 15, which is favourable when introducing the catheter.

The distal end 3 of the catheter 1 is introduced into a blood vessel 18 of a patient inside of which the stent 13 is to be implanted. The embodiment of the catheter according to the invention shown here is provided with the balloon 9 for dilating a narrowed blood vessel 18, in addition to the balloon 10 designed for expanding the stent 13. By employing the two balloons 9, 10, dilating the vessel and implanting the stent can be achieved using one and the same catheter 1, without it being necessary to change catheters.

With the treatment the catheter 1 is first introduced into the blood vessel 18 to such a degree that the first balloon 9 is positioned at the stenosed section which is to be dilated. Subsequently a medium under pressure is supplied via the connecting member 7 through the lumen 17 of the inner tube-like element 6. Via the opening 21 in the wall of the inner tube-like member this medium under pressure flows into the balloon 9 as a result of which it will expand. Consequently the blood vessel 18 will be dilated (fig. 3).

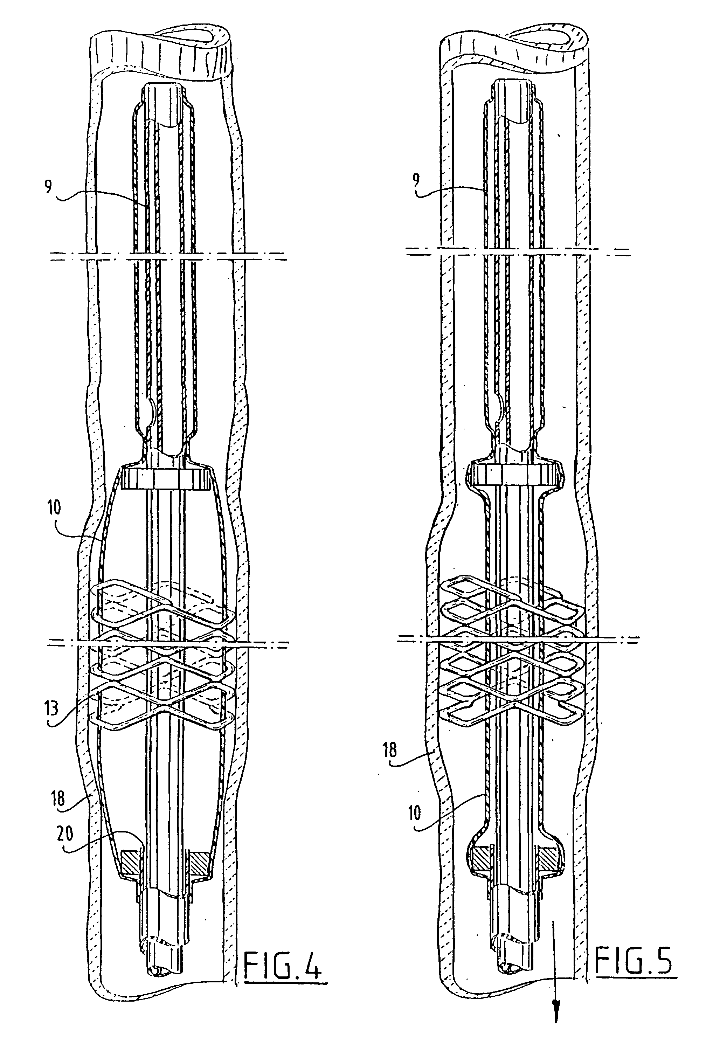

Next the pressure inside the balloon 9 will be reduced, as a result of which it will resume its original shape. The catheter is advanced further into the vessel so that the balloon 10 will be situated at the dilated section of the vessel. By supplying medium under pressure to balloon 10 via connector 8 and the interspace between the tube-like elements 5, 6, which interspace is connected with the inside of the balloon 10 at the end of the outer tube-like element 5 as a ring-shaped opening 20, this balloon 10, and hence the stent 13, will be expanded. This situation has been illustrated in figure 4.

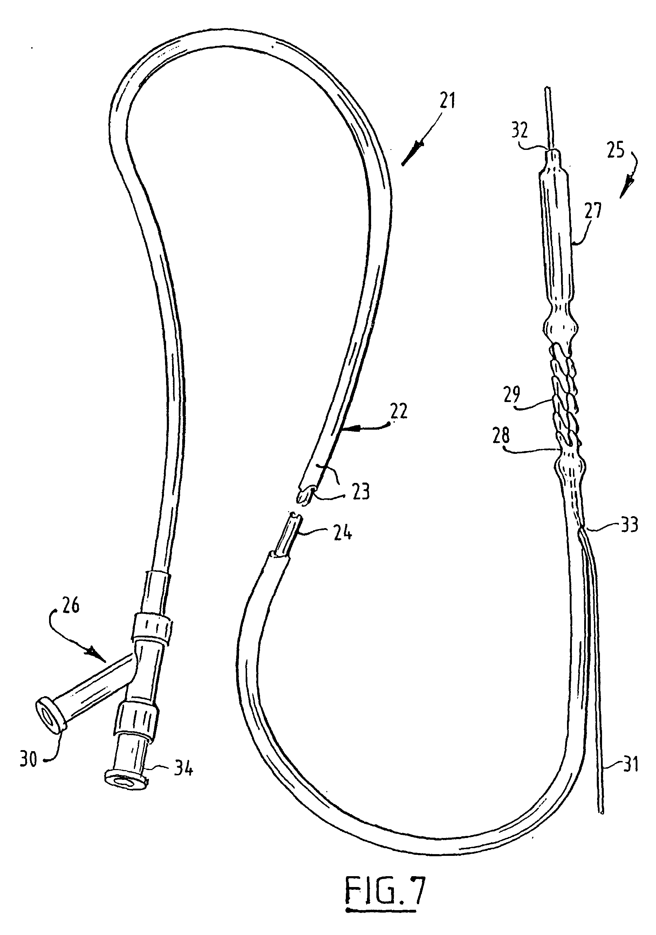

Subsequently one allows the pressure in the balloon 10 to fall, as a result of which the balloon 10 will resume its original shape with small diameter as shown in figure 5. The catheter can now be withdrawn, whereby as is shown in figure 6, the stent 13 remains behind in the blood vessel 18, in order to support the wall thereof.

The catheter and the accompanying stent shown in the figures only represent examples of embodiments. Other embodiments of stents known as such, can be used in conjunction with the catheter according to the invention. Because of the good axial fixation of the stent, the latter will remain positioned in a reliable manner at the site of the balloon used for the purpose of expansion, so that it is not necessary to use a guiding catheter or a sheath.

The catheter according to the invention may comprise any number of balloon-shaped members, of which at least one comprises bulges on either side to retain a stent.

In the embodiment shown, the rings 14 and 15 have been made of material which is clearly visible when subjected to X-rays, so that the position of these rings 14, 15 and consequently the position of the stent, placed in between these bulges, can be rendered clearly visible in a catheterization laboratory.

The catheter 21 shown in figure 7 corresponds for the major part with the catheter 1 of figure 1. This catheter 21 thus comprises a tube-like basic body 22 with a distal end 25 and a proximal end 26. At the distal end 25 two balloons 27, 28 have been arranged. The balloon 27 is for dilating a narrowed section of a blood vessel. Around the balloon 28, a stent 29 has been arranged in compressed state. This stent 29 can be expanded, as described before, by means of the balloon 28 and thus be implanted in the section of the vessel dilated beforehand by means of the balloon 27.

With the embodiment shown, the basic body 22 is made up of an outer tube-like element 23 and an inner tube-like element 24. A lumen inside the inner tube-like element 24 has at the proximal end of the catheter 21 been connected to a connection 34, and at the distal end to the inside of the balloon 27 in order to convey medium under pressure from the connection 34 to the balloon 27 in order to expand the latter. In between the inner tube-like element 24 and the outer tube-like element 23, a in cross-section circular lumen is formed which, at the proximal end 26 of the catheter, is connected to the connection 30 and at the distal end 25 to the inside of the balloon 28 in order to be able to expand the latter in a similar manner.

For introducing the catheter into a patient a guide wire 31 is employed in the usual manner. For the purpose of receiving this guide wire 31 a lumen 36 has been formed in the catheter 21 which extends from an end hole 32 to a wall hole 33 in the catheter. This wall hole has been arranged in the wall of the basic body 22 at a position in between the distal and the proximal end.

With the preferred embodiment shown in figure 7, the wall hole has been arranged close to the relatively proximal active element of the catheter 21, the balloon 28. The lumen 36 for receiving the guide wire 31 only extends therefore through the end-section of the catheter 21.

## Claims

1. Catheter (1) comprising a tube-like body (4) with a proximal (2) and a distal (3) end, at least one balloon member (10) arranged close to the distal end (3) which is connected via a lumen (17) in the basic body (4) to a connecting member at the proximal end (3), wherein close to either end of the balloon member a ring (14,15) has been arranged to the basic body (4) and wherein a compressed stent (13) has been arranged around the balloon member (10) and in between the rings (14,15), **characterized by**, the rings (14,15) being arranged inside the balloon-shaped member (10).

2. Catheter as claimed in claim 1, wherein the outside diameter of the compressed stent (13) is smaller than the outside diameter of the catheter (1) at the site of the rings (14,15).

3. Catheter as claimed in claim 1 or 2, wherein each ring (14,15) has been made of material visible when subjected to X-rays and/or when used in conjunction with an NMR device.

4. Catheter as claimed in one of the previous claims, comprising two successive balloon members (9,10), wherein the rings (14,15) and the stent (13) are arranged at the site of the relatively proximal balloon member (10).

5. Catheter as claimed in claim 4, wherein the basic body (2) comprises an outer tube-like element (5) with a lumen inside of which an inner tube-like element (6) has been received, a lumen (17) of the inner tube-like element has been connected with the relatively distal balloon member (9) and the interspace between the inner tube-like member and the outer tube-like member is connected with the comparatively proximal balloon-shaped member (10).

## Patentansprüche

1. Katheter (1), aufweisend einen rohrartigen Körper (4) mit einem proximalen (2) und einem distalen (3) Ende und zumindest ein Ballonteil (10), das in der Nähe des distalen Endes (3) angeordnet ist, welches über ein Lumen (17) in dem Basiskörper (4) mit einem Verbindungsteil an dem proximalen Ende (3) verbunden ist, wobei in der Nähe jedes Endes des Ballonteils ein Ring (14, 15) an dem Basiskörper (4) angeordnet wurde, und wobei ein zusammengedrückter Stent (13) um das Ballonteil (10) herum und zwischen den Ringen (14, 15) angeordnet wurde, **dadurch gekennzeichnet, dass** die Ringe (14, 15) innerhalb des ballonförmigen Teils (10) angeordnet sind.

2. Katheter nach Anspruch 1, wobei der Außendurchmesser des zusammengedrückten Stents (13) kleiner als der Außendurchmesser des Katheters (1) an der Stelle der Ringe (14, 15) ist.

3. Katheter nach Anspruch 1 oder 2, wobei jeder Ring (14, 15) aus Material hergestellt wurde, das sichtbar ist, wenn es Röntgenstrahlen ausgesetzt ist und/oder wenn es in Verbindung mit einer NMR-Vorrichtung benutzt wird.

4. Katheter nach einem der vorhergehenden Ansprüche, aufweisend zwei aufeinander folgende Ballonteile (9, 10), wobei die Ringe (14, 15) und der Stent (13) an der Stelle des relativen proximalen Ballonteils (10) angeordnet sind.

5. Katheter nach Anspruch 4, wobei der Basiskörper (2) ein äußeres rohrartiges Element (5) mit einem Lumen aufweist, in welchem ein inneres rohrartiges Element (6) aufgenommen wurde, ein Lumen (17) des inneren rohrartigen Elements mit dem relativen distalen Ballonteil (9) verbunden wurde, und der Zwischenraum zwischen dem inneren rohrartigen Teil und dem äußeren rohrartigen Teil mit dem vergleichsweise proximalen ballonförmigen Teil (10) verbunden ist.

## Revendications

1. Cathéter (1) comprenant un élément en forme de tube (4) avec une extrémité proximale (2) et distale (3), au moins un élément à ballonnet (10) disposé à proximité de l'extrémité distale (3) qui est raccordé via une lumière (17) dans le corps de base (4) à un élément de raccordement au niveau de l'extrémité proximale (3), dans lequel on a disposé une bague (14, 15) à proximité de chacune des extrémités de l'élément à ballonnet sur le corps principal (4), et un stent comprimé (13) autour de l'élément à ballonnet (10) et entre les bagues (14, 15), **caractérisé en ce que** les bagues (14, 15) sont disposées à l'intérieur de l'élément en forme de ballonnet (10).

2. Cathéter selon la revendication 1, dans lequel le diamètre extérieur du stent (13) comprimé est inférieur au diamètre extérieur du cathéter (1) au niveau des bagues (14, 15).

3. Cathéter selon la revendication 1 ou 2, dans lequel chaque bague (14, 15) est fabriquée dans un matériau visible aux rayons X et/ou lorsqu'il est utilisé en conjonction avec un appareil de RMN.

4. Cathéter selon l'une quelconque des revendications précédentes, comprenant deux éléments à ballonnets successifs (9, 10), dans lequel les bagues (14, 15) et le stent (13) sont disposés au niveau de l'élément à ballonnet relativement proximal (10).

5. Cathéter selon la revendication 4, dans lequel le corps de base (2) comprend un élément extérieur en forme de tube (5) avec une lumière à l'intérieur de laquelle un élément extérieur en forme de tube (6) a été reçu, une lumière (17) de l'élément intérieur en forme de tube a été raccordée avec l'élément à ballonnet relativement distal (9) et l'espacement entre l'élément intérieur en forme de tube et l'élément extérieur en forme de tube est raccordé avec l'élément à ballonnet comparativement proximal (10).
